# EUROPÄISCHE PATENTSCHRIFT

(11) **EP 0 959 917 B1**
(45) Veröffentlichungstag und Bekanntmachung des Hinweises auf die Patenterteilung: **19.09.2007**
(21) Anmeldenummer: 97914183.5
(22) Anmeldetag: 28.02.1997
(51) Int. Cl.: A61M 5/142

(54) **VERFAHREN UND VORRICHTUNG ZUM ENDOSKOPISCHEN INJIZIEREN MINDESTENS EINER FLÜSSIGKEIT**
METHOD AND DEVICE FOR ENDOSCOPIC INJECTION OF AT LEAST ONE FLUID
PROCEDE ET DISPOSITIF POUR L'INJECTION ENDOSCOPIQUE D'AU MOINS UN LIQUIDE

(30) Priorität: 01.03.1996 DE 19607922
(43) Veröffentlichungstag der Anmeldung: 01.12.1999
(73) Patentinhaber: Grund, Karl Ernst, 72070 Tübingen (DE)
(72) Erfinder: GRUND, Karl, Ernst, D-72070 Tübingen (DE); FARIN, Günter, D-72070 Tübingen (DE); ÖZMEN, Dogan, D-58642 Iserlohn (DE)
(74) Vertreter: Bohnenberger, Johannes
(86) Internationale Anmeldenummer: PCT/EP1997/001039
(87) Internationale Veröffentlichungsnummer: WO 1997/031667

(56) Entgegenhaltungen:
- EP-A- 0 398 583
- WO-A-96/36381
- US-A- 5 116 313
- DATABASE WPI Section PQ, Week 8209 14.April 1981 Derwent Publications Ltd., London, GB; Class P31, AN 82-c3916e XP002035724 : & SU 831 114 A (ROST MED INST) , 25.Mai 1981

## Beschreibung

Die Erfindung betrifft eine Vorrichtung zum endoskopischen Injizieren mindestens einer Flüssigkeit.

Aus der US-A-5 116 313 ist eine nadellose Injektionsvorrichtung bekannt, bei welcher mittels einer Punktvorrichtung nach einem ersten, kräftigeren "Strahl" eine definierte Flüssigkeitsmenge mit geringerem Druck in das vorgeformte Loch eingespritzt wird. Weitere Anwendungen der bekannten Vorrichtung sind in der Druckschrift nicht vorgeschlagen.

Bei endoskopischen Operationen werden in natürlichen oder künstlichen Körperhöhlen verschiedenste Manipulationen vorgenommen, wobei es immer wieder darauf ankommt, Gewebe zu entfernen und/oder Blutungen zu stillen. Hierbei wird unter anderem mit Injektionstechniken gearbeitet. Beispielsweise kann man Polypen, die auf einem hinreichend langen Stiel sitzen, relativ einfach (mittels einer Schlinge) entfernen, jedoch ist dies nicht möglich, wenn es sich um sogenannte sessile Polypen anzuheben, indem man in das darunterliegende Gewebe Gewebe Flüssigkeit, insbesondere physiologische Kochsalzlösung mittels einer Injektionsnadel spritzt. Der so angehobene sessile Polyp kann dann wiederum mit den bekannten Techniken entfernt werden.

Ein anderer Fall, in welchem Flüssigkeiten zu injizieren sind, liegt dann vor, wenn Blutungen gestillt werden sollen. Zum einen geschieht eine solche Stillung von Blutungen dadurch, daß durch Injizieren von phyiologischer Kochsalzlösung und Bilden von Depots rings um ein Blutgefäß dieses mechanisch verschlossen wird, zum anderen kann durch Injektion von Medikamenten, insbesondere von Vasokonstriktiva, eine entsprechende Wirkung erzielt werden. Eine andere Methode, Blutungen zu stillen, liegt in der Injektion von Fibrinklebern.

Schließlich soll noch eine weitere Anwendung von Injektionen erwähnt werden, und zwar die Markierung von Operations- oder Behandlungsstellen. Es muß nämlich oftmals nach einer Behandlung, z. B. nach dem Entfernen einer Wucherung im Darm, diese Stelle in regelmäßigen Abständen wieder untersucht werden, um festzustellen, wie das Behandlungsergebnis war bzw. wie die Heilung verläuft. Insbesondere bei kleinflächigen Operationsstellen und gut verlaufenden Heilungen ist es sehr schwer, die behandelten Stellen wiederzufinden. Zu diesem Zweck ist es sinnvoll, die behandelte Stelle einzufärben bzw. Markierungspunkte in ihre Nähe zu setzen.

Der Erfindung liegt die Aufgabe zugrunde, eine Vorrichtung aufzuzeigen, mittels derer bei endoskopischen Operationen Flüssigkeiten injiziert werden können.

Es wird zur Lösung der eingangs genannten Probleme eine Vorrichtung aufgezeigt zum endoskopischen Injizieren mindestens einer Flüssigkeit, insbesondere einer Lösung, einer Suspension oder einer Emulsion oder einer Mischung mehrerer Lösungen, in ein Gewebe in einem Hohlorgan, einer Körperhöhle oder in einer künstlichen Höhle in einem menschlichen oder tierischen Körper. Hierbei wird eine definierte Flüssigkeitsmenge aus einem Schlauchende oder einer Düse mit definiert einstellbaren hydraulischen Parametern wie Druck, Druckanstiegsgeschwindigkeit, Dauer und Geschwindigkeit der ausgestoßenen Flüssigkeit und mit solcher Engergie ausgestoßen, daß die Flüssigkeit nach im wesentlichen frei fliegendem Durchqueren einer Wegstrecke zwischen dem Schlauchende oder der Düse und dem Gewebe in das Gewebe eindringt. Es wird also beim erfindungsgemäßen Verfahren (bzw. bei der erfindungsgemäßen Vorrichtung) keine Injektionsnadel verwendet, die in die Gewebestelle eingestochen werden müßte, an welcher man die Flüssigkeit appliziert haben will. Dadurch ist die Eignung des erfindungsgemäßen Verfahrens zum Applizieren von Flüssigkeiten bei endoskopischen Operationen besonders gut. Insbesondere kann durch die entsprechende Einstellung der hydraulischen Parameter gewährleistet werden, daß die Flüssigkeit bis zu einer definierten Tiefe in das Gewebe eindringt, in der die Flüssigkeit dann die gewünschte Wirkung entfalten kann. Dadurch kann auch eine erheblich vergrößerte Sicherheit gegenüber ungewollten Wirkungen erzielt werden. Dann z. B., wenn man eine Stelle im Darm markieren will, ist es außerordentlich gefährlich, mit einer Injektionsnadel zu arbeiten, da die Darmwand sehr leicht durchstochen werden kann. Bei Anwendung des erfindungsgemäßen Verfahrens ist diese Gefahr erheblich geringer.

Vorzugsweise geht man so vor, daß man nicht die gesamte zu injizierende Flüssigkeitsmenge in einer "Portion" appliziert. Man injiziert vielmehr die gewünschte Flüssigkeitsmenge in einer Vielzahl von im wesentlichen gleich großen Minimalmengen. Dadurch ist einerseits gewährleistet, daß im wesentlichen jede Flüssigkeitsmenge injiziert werden kann, andererseits aber die hydraulischen Parameter, also diejenigen Parameter, welche die Injektionstiefe, den Durchmesser der im Gewebe sich ausbildenden Öffnung usw. bestimmen, bei einem konstanten Wert gehalten werden können, so daß das Injektionsergebnis optimal ist.

Vorzugsweise wird die Flüssigkeit nicht senkrecht in das Gewebe (bzw. senkrecht zu dessen Oberfläche) injiziert, sondern unter einem Winkel. Dieser Winkel beträgt vorzugsweise 30° bis 60° zum Gewebe. Überraschenderweise haben sich dadurch die besten Injektionsergebnisse erzielen lassen.

Die Flüssigkeit wird nach Möglichkeit in einem derart dünnen Strahl injiziert, daß sich das beim Injizieren entstehende Loch im Gewebe nach dem Injizieren sofort wieder schließt, so daß praktisch keine injizierte Flüssigkeit wieder entlassen wird. Dies kann ohne weiteres bewerkstelligt werden, da durch eine entsprechend große Anzahl (an sich kleiner) Einzelportionen, die gewünschte Flüssigkeitsmenge trotz eines sehr dünnen Flüssigkeitsstrahls injizierbar ist.

Wenn man zwei verschiedene, miteinander reagierende Flüssigkeiten, wie z. B. den eingangs genannten Fibrinkleber oder dergleichen applizieren will, werden die Flüssigkeiten vorzugsweise erst kurz vor einem gemeinsamen Injizieren vermischt oder aber derart kurz nacheinander (in den genannten Einzelportionen) an derselben Stelle injiziert, daß sie sich am Injektionsort vermischen. Besonders bei Flüssigkeitsgemischen, bei denen die Einzel-Flüssigkeiten sehr schnell miteinander reagieren, insbesondere sich verfestigen oder verkleben, ist diese Injektion von Flüssigkeiten nacheinander ein hervorragendes Mittel, um eine Reaktion in der eigentlichen Injektionsvorrichtung (Düse oder Schlauchende) und damit eine Zerstörung der Apparatur zu verhindern.

Ein gewisses Problem kann darin liegen, daß unbeabsichtigt Flüssigkeit aus dem Schlauchende austritt. Dies spielt z. B. dann keine Rolle, wenn die Flüssigkeit eine physiologische Kochsalzlösung zum Unterspritzen eines Polypen ist. Dann aber, wenn die Flüssigkeit eine Markierungsflüssigkeit, also eine Tätowierungstinte ist, hat ein versehentliches Austreten der Flüssigkeit aus dem Schlauchende fatale Folgen. Es wird nämlich eine Stelle markiert, die überhaupt nicht markiert werden soll. Ähnlich fatal wäre das versehentliche Austreten von Medikamenten. Dies läßt sich aber schon dadurch nicht vermeiden, daß ein bis zum distalen Ende gefüllter Schlauch, der im Arbeitskanal des Endoskops steckt, sein Volumen bei der Bewegung des Endoskops - wenn auch geringfügig - ändert, so daß Flüssigkeit austritt. Es wird nun vorgeschlagen, aus dem Schlauchende unbeabsichtigt austretende Flüssigkeit abzusaugen. Dies wird dann besonders einfach, wenn man einen ständigen Absaugstrom erzeugt, dessen Leistung hinsichtlich Absäugvolumen pro Zeiteinheit aber geringer ist als die Injektionsleistung hinsichtlich ihres Injektionsvolumens pro Zeiteinheit. Es wird dadurch sichergestellt, daß die langsam unbeabsichtigt austretende Flüssigkeit vollständig abgesaugt wird, die mit hoher Geschwindigkeit, also relativ hohem Volumenstrom pro Zeiteinheit austretende Flüssigkeit aber praktisch nicht vermindert wird.

Die erfindungsgemäße endoskopische Injektionsvorrichtung, die zum Injizieren mindestens einer Flüssigkeit, insbesondere einer Lösung, einer Suspension oder einer Emulsion sowie Mischungen hiervon in ein Gewebe in einem Hohlorgan, einer Körperhöhle oder einer künstlichen Höhle in einem menschlichen oder tierischen Körper dient, umfaßt einen Schlauch mit einem distalen sowie einem proximalen Ende, der durch einen Arbeitskanal eines Endoskops derart hindurchführbar oder in ein Endoskop derart eingebaut ist, daß das distale Schlauchende aus einem distalen Ende des Endoskops hervortritt oder aber im wesentlichen beim Endoskopende ebenfalls endet. Es ist eine Pumpeinrichtung vorgesehen, die mit dem proximalen Schlauchende in dichter Verbindung steht und derart ausgebildet ist, daß sie auf ein Steuersignal hin eine definierte Flüssigkeitsmenge mit definiert einstellbaren hydraulischen Parametern wie Druck, Druckanstiegsgeschwindigkeit, Dauer und/oder Geschwindigkeit aus dem distalen Schlauchende mit einer solchen Energie ausstößt, daß die Flüssigkeit in das Gewebe eindringt.

Das distale Ende des Schlauches ist hierbei vorzugsweise derart geformt oder mit einer Düse derart versehen, daß sich das im Gewebe beim Injizieren entstehende Loch schließt, ohne im wesentlichen die injizierte Flüssigkeit zuvor zu entlassen. Dies bedeutet, daß der Durchmesser des Schlauchendes bzw. der Düse vorzugsweise sehr gering ist.

Die Pumpeinrichtung ist vorzugsweise derart ausgebildet, daß eine gewünschte Flüssigkeitsmenge in einer Vielzahl von nacheinander injizierten kleinen Einzelvolumina injizierbar ist. Es kommt also darauf an, daß die Pumpeinrichtung sehr kleine Flüssigkeitsvolumina in relativ kurzer Zeit nacheinander ausstoßen kann.

Vorzugsweise sind am distalen Ende des Schlauches Ziel- und/oder Positioniereinrichtungen vorgesehen, um das distale Ende in einem vorbestimmten Abstand vom Gewebe und/oder in einer vorbestimmten Richtung zum Gewebe zu halten. Hier sind eine Vielzahl von Zielvorrichtungen vorstellbar. Vorzugsweise sind diese Ziel- und/oder Positioniervorrichtungen derart ausgebildet, daß die Flüssigkeit unter einem Winkel von etwa 30° bis 60° in das Gewebe injizierbar ist. Es kommt also darauf an, die Zielvorrichtungen für diesen Fall so auszubilden, daß der die Vorrichtung Handhabende abschätzen kann, unter welchem Winkel die Düse bzw. das Schlauchende auf die Gewebeoberfläche gerichtet ist.

Für besondere Anwendungszwecke weist der Schlauch mindestens in seinem distalen Abschnitt mehrere, insbesondere zwei Lumina auf. Die Pumpeinrichtungen umfassen mehrere Einzelpumpen, um mehrere, insbesondere zwei Flüssigkeiten zu deren Vermischung zu injizieren. Dies ist z. B. dann angebracht, wenn Zwei-Komponenten-Kleber, z. B. Fibrinkleber, injiziert werden soll.

Bei einer weiteren Ausführungsform der Erfindung umfaßt der Schlauch vorzugsweise ein Gas- und/oder Flüssigkeitsrückführungslumen, das derart beim distalen Ende in den Schlauch mündet, daß im Schlauch enthaltene Flüssigkeit am Austreten aus dem distalen Ende gehindert wird. Hierzu ist es von Vorteil, wenn eine Saugeinrichtung zum Absaugen von Flüssigkeit aus dem Flüssigkeitrückführungslumen vorgesehen ist, die im wesentlichen ständig in Betrieb ist. Diese Saugeinrichtung kann beispielsweise eine in einem Operationssaal oftmals vorhandene Absaugvorrichtung sein.

Bei einer bevorzugten Ausführungsform umfaßt der Schlauch bei oder an seinem distalen Ende einen einlumigen Abschnitt oder eine Düse, was sowohl das Führen von eventuell austretender Flüssigkeit erleichtert, als auch beim Ausstoßen von zwei verschiedenen Flüssigkeit deren Vermischung noch im Schlauch bzw. in der Düse ermöglicht.

Der Schlauch ist vorzugsweise aus einem derartigen Material und derart drucksteif ausgebildet und/oder mit Versteifungseinrichtungen derart versehen, daß ein in sein proximales Ende eingeführter Druckpuls im wesentlichen ungedämpft oder unverändert aus seinem distalen Ende austritt. Wichtig ist hierbei auch ein Material zu wählen, das eine geringe innere Dämpfung hat. Man kann den Schlauch nun derart gestalten, daß er einen Druckpuls in seinem Resonanzbereich ähnlich einem Wellenleiter hindurchläßt. Erstaunlicherweise konnte gezeigt werden, daß ein Schlauch mit einem Lumen-Durchmesser von etwa 0,2mm einen derartigen Druckpuls im wesentlichen ungedämpft über eine Länge von etwa 2m mit hinreichender Anstiegsgeschwindigkeit und hinreichendem Druck hindurchläßt.

Vorzugsweise wird der Schlauch mit gegebenenfalls vorgesehener Düse zusammen mit einem Flüssigkeitsreservoir zum Speichern größerer, während der Dauer einer längerer Anwendung oder Operation zu injizierender Flüssigkeitsmenge als einheitlich handhabbare, sterile Injektionseinheit ausgebildet. Dadurch ist es möglich, mit wenigen Handgriffen und in kürzester Zeit auch dann während einer endoskopischen Operation eine Injektion vorzubereiten und durchzuführen, wenn dies zunächst gar nicht beabsichtigt war. Hierbei ist es von besonderem Vorteil, wenn man die Injektionseinheit als nur einmal zu verwendendes Teil ausbildet, so daß sichergestellt wird, daß keine Abnutzungserscheinungen (Verstopfungen und dergleichen) auftreten und auch keine unsterilen Einrichtungen Verwendung finden.

Vorzugsweise umfaßt die Injektionseinheit die Pumpeinrichtung zumindest teilweise. Eine Möglichkeit besteht darin, die Pumpeinrichtung mit einer Membran, einem Kolben oder einem derart bewegbaren Element als Druckerzeugungsteil auszustatten, welches das Flüssigkeitsreservoir oder ein Zwischen-Reservoir im Schlauch dicht abschließt. Zum Bewegen der Membran bzw. zum Erzeugen des Drucks kann eine ebenfalls mit der Gesamteinrichtung fest verbundene (Einmal-) Vorrichtung vorzugsweise aber eine gesonderte, wiederverwendbare Antriebseinrichtung mit vorgesehen sein, welche die Kolben oder die Membran mit einer einstellbaren Pumpkraft beaufschlagt. Hierzu ist ein entsprechender Impulsgenerator vorgesehen.

Bevorzugte Ausführungsformen der Erfindung ergeben sich aus den Unteransprüchen.

Nachfolgend wird die Erfindung anhand von Abbildungen näher erläutert. Hierbei zeigen:
- Fig. 1.1 bis 1.7: verschiedene Ausführungsformen von Schlauchenden;
- Fig. 2: eine schematisierte Darstellung einer Pumpeinrichtung mit Steuerschaltung;
- Fig. 3: einen schematisierten Längsschnitt einer anderen bevorzugten Ausführungsform einer Injektionseinrichtung samt Pumpeinrichtung;
- Fig. 4: ein Schnitt entlang der Linie IV-IV aus Fig. 3;
- Fig. 5: eine Antriebseinrichtung für die Pumpeinrichtung nach den Fig. 3 und 4;
- Fig. 6: eine weitere Ausführungsform einer Antriebseinrichung für die Pumpeinrichtung nach den Fig. 3 und 4; und
- Fig. 7: eine schematisierte Darstellung einer weiteren Ausführungsform der Injektionseinrichtung.

Bei der nachfolgenden Beschreibung werden für gleiche und gleichwirkende Teile dieselben Bezugsziffern verwendet.

Bei der hier dargestellten Vorrichtung dreht es sich um die Injektion von Flüssigkeiten verschiedenster Art während endoskopischer Operationen. Ein Hauptproblem bei derartigen Injektionen liegt darin, daß die Manipulation schwierig und aufwendig ist, insbesondere aber ein hohes Maß an Erfahrung fordert. Wenn man mittels einer Nadel injiziert, so muß nicht nur der Punkt auf der Gewebeoberfläche gefunden werden, in welchen die Flüssigkeit (Markierungsflüssigkeit, Medikament, Kochsalzlösung, usw.) appliziert werden soll, es muß auch noch die Nadel in eine bestimmte Tiefe eingestochen werden. Erst danach folgt das eigentliche Applizieren der Flüssigkeit, was wiederum Fingerspitzengefühl erfordert. Dies bedeutet, daß neben dem zweidimensionalen "Zielen" noch die dritte Dimension (Einstichtiefe) sowie die Injektionsmenge zu kontrollieren sind.

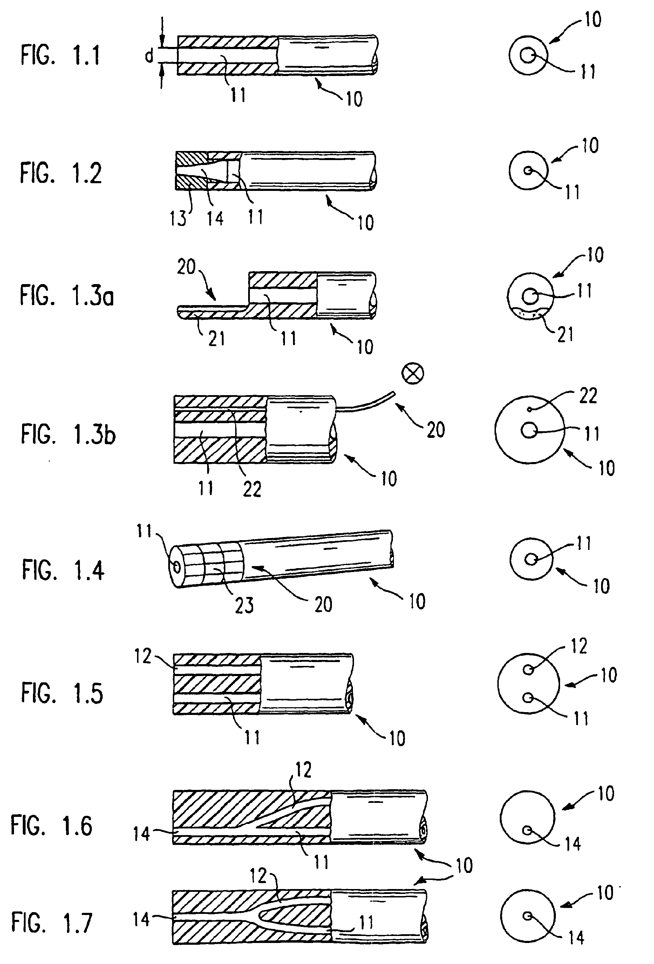

Verwendet man die erfindungsgemäße Vorrichtung, so kann mittels einer Pumpvorrichtung durch einen in Fig. 1.1 gezeigten Schlauch 10 bzw. dessen Lumen 11 die Flüssigkeit mit hinreichend hoher Geschwindigkeit derart ausgestoßen werden, daß sie in das Gewebe eindringt. Die Eindringtiefe wiederum hängt davon ab, wie groß der Durchmesser d des Lumens 11 des Schlauches 10 ist und mit welcher Geschwindigkeit die Flüssigkeit ausgestoßen wird. Andere Parameter, wie beispielsweise der Abstand des Schlauches 10 von der Gewebeoberfläche, spielen auch noch eine Rolle.

Bei der in Fig. 1.2 gezeigten Variante ist im Ende des Schlauches 10 eine Düse 13 vorgesehen, die einen Endabschnitt 14 des Lumens 11 des Schlauches 10 bildet, dessen Durchmesser in Richtung auf die Öffnung im wesentlichen exponentiell sinkt. Durch diese besondere Steigung ist es möglich, den durch das Lumen 11 ankommenden Druckstoß im wesentlichen verlustlos zu "konzentrieren", also eine Geschwindigkeiterhöhung der strömenden Flüssigkeit zu bewirken.

Bei der in Fig. 1.3a gezeigten Ausführungsform der Erfindung weist der Schlauch 10 an seinem Ende eine Zieleinrichtung 20 auf, die hier als "Abstandsfinger" ausgebildet ist. Mittels dieses Fingers kann der Benutzer nicht nur den Abstand des Schlauches 10 von der Gewebeoberfläche besser beurteilen, er kann auch die Stelle besser abschätzen, an welcher die ausgestoßene Flüssigkeit voraussichtlich auftrifft.

Bei der in Fig. 1.3b gezeigten weiteren Ausführungsform der Erfindung ist in den Schlauch 10 ein Lichtleiter 22 mit eingebaut, der die Zieleinrichtung 20 bildet. Der Lichtleiter 22 weist hierbei eine derart ausgebildete Endfläche auf, daß das aus ihm austretende (Laser-) Licht im wesentlichen geradlinig als "Lichtzeiger" abgestrahlt wird, dessen Auftreffort auf der Gewebeoberfläche im wesentlichen mit dem Ort übereinstimmt, in welchen dann die Flüssigkeit injiziert wird.

Bei der in Fig. 1.4 gezeigten Ausführungsform der Erfindung umfaßt die Zieleinrichtung 20 Markierungen 23 auf dem Endabschnitt des Schlauches 10. Diese Markierungen 23 sind im Blickfeld der Endoskop-Optik und erleichtern es dem Benutzer Entfernungs- und/oder Richtungsabschätzungen vorzunehmen. Selbstverständlich sind die genannten Maßnahmen auch miteinander kombinierbar.

Bei der in Fig. 1.5 gezeigten Ausführungsform der Erfindung ist der Schlauch 10 mit zwei Lumina 11 und 12 ausgebildet. Diese zwei Lumina 11 und 12 können nun sowohl dazu dienen, zwei verschiedene Flüssigkeiten zu injizieren - dann stehen sie mit zwei gleichartigen Pumpeinrichtungen in Verbindung - oder aber das eine Lumen 11 zum Injizieren, das andere Lumen 12 zum Absaugen zu verwenden. Selbstverständlich ist es auch möglich, das eine Lumen zum Injizieren von Flüssigkeiten, das andere Lumen aber zum Spülen zu verwenden.

Bei der in Fig. 1.6 gezeigten Ausführungsform der Erfindung sind wieder zwei Lumina 11 und 12 im Schlauch 10 vorgesehen, jedoch münden diese in einen gemeinsamen Endabschnitt 14. Diese Anordnung dient nun bevorzugterweise entweder dazu, bei gemeinsamem Ausstoßen von Flüssigkeit durch die Lumina 11 und 12 zum Zwecke des Injizierens diese Flüssigkeit sich schon im Endabschnitt 14 vermischen zu lassen. Es ist allerdings auch möglich, durch die Lumina 11 und 12 sehr schnell hintereinander Einzelquanten von Flüssigkeit abzugeben, die dann nacheinander durch den Endabschnitt 14 hindurch ausgestoßen werden und in dasselben Loch im Gewebe eintreten, so daß sich die Flüssigkeiten im Gewebe vermischen.

Die in Fig. 1.7 gezeigte Ausführungsform unterscheidet sich von der nach Fig. 1.6 lediglich dadurch, daß der Endabschnitt 14 symetrisch zu den beiden Lumina 11 und 12 angeordnet ist. Sowohl diese Ausführungsform als auch die anhand von Fig. 1.6 beschriebene Ausführungsform eignen sich weiterhin dazu, das eine Lumen 11 zum Injizieren von Flüssigkeiten, das andere Lumen 12 jedoch zum Absaugen zu verwenden. Wenn man nämlich beispielsweise an einem Operationsfeld eine Markierung anbringen will, eine Tätowierungsmarke setzen will, so darf die Tätowierungsflüssigkeit in keinem Fall ungewollt austreten. Andererseits muß das Lumen 11 des Schlauches 10 im wesentlichen vollständig gefüllt sein, will man einen zum Injizieren geeigneten Druckstoß durch den Schlauch 10 hindurchschicken, der ja oftmals eine nicht unbeträchtliche Länge (1 bis 2m) aufweist. Um also den Schlauch zu füllen, läßt man durch das eine Lumen 11 zunächst langsam Flüssigkeit strömen und schließt das proximale Ende des zweiten Lumens 12 an eine Unterdruckquelle an. Sobald die Flüssigkeit aus dem zweiten Lumen 12 am proximalen Ende austritt, weiß man, daß der Schlauch hinreichend gefüllt ist. Man kann dann weitersaugen, so daß Luft (oder auch Flüssigkeit) vom Operationsort in das distale Ende 14 eintritt und durch das Lumen 12 abgesaugt wird. Das Lumen 11 bleibt dennoch gefüllt, da ja keine Flüssigkeit nachströmt. Sobald dann ein Druckstoß auf das proximale Ende des Lumens 11 gegeben wird, "schießt" eine entsprechende Flüssigkeitsportion aus dem distalen Ende 14 des Schlauches 10 heraus und dringt in das Gewebe ein.

Es ist nun von besonderem Vorteil, wenn die gesamte Anordnung als einzeln handhabbares Teil derart ausgebildet ist, daß die zu applizierende Flüssigkeit in einer Pumpeinrichtung gespeichert ist und die Pumpeinrichtung mit dem Schlauch fest verbunden ist. Die gesamte Anordnung ist weiterhin steril. Auf diese Weise ist es möglich, sehr schnell dann eine Injektion vorzunehmen, wenn sie nötig ist, ohne daß für jede Operation besondere Vorbereitungsmaßnahmen zu treffen wären. Es entspricht die Anordnung sozusagen einer bereits gefüllten Einmalspritze.

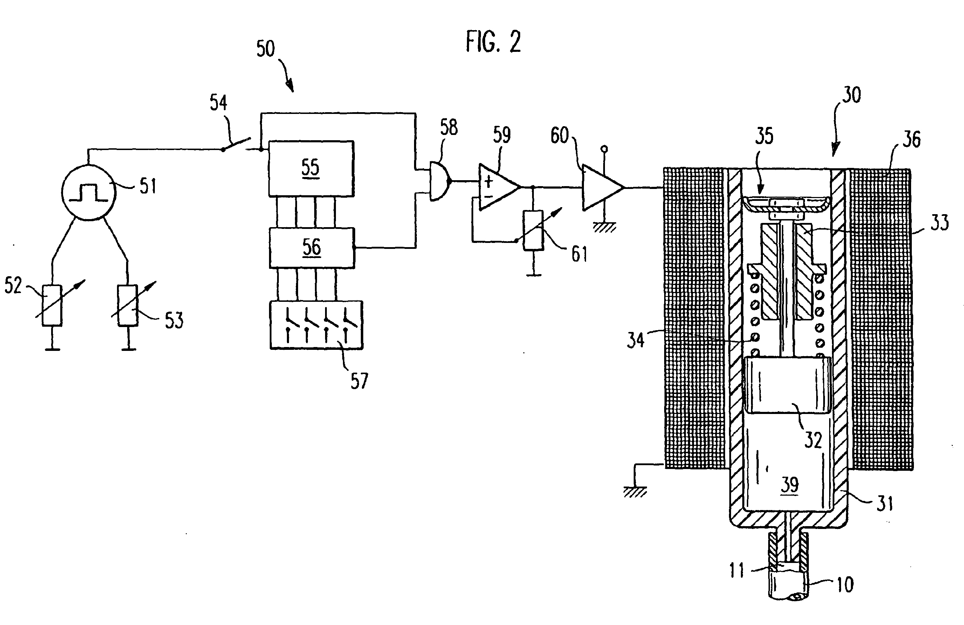

Die in Fig. 2 gezeigte Ausführungsform der Erfindung umfaßt zu diesem Zweck einen Zylinder 31, an welchen der Schlauch 10 angeschlossen ist und der in seinem Inneren ein Flüssigkeitsreservoir 39 aufweist, das einerseits mit dem Lumen 11 des Schlauches 10 kommunizierend verbunden, andererseits durch einen Kolben 32 abgeschlossen ist.

Der Kolben 32 weist an seinem hinteren, dem Schlauch 10 abgewandten Ende einen verjüngten Abschnitt auf, auf welchem ein als zylindrische Hülse ausgebildeter Hammer 33 axial verschieblich entgegen der Kraft einer Feder 34 derart angeordnet ist, daß der Hammer 33 entgegen der Kraft der Feder 34 beschleunigt werden kann und auf den verdickten Teil des Kolbens 32 trifft, so daß diesem ein Vorwärtsimpuls mitgeteilt wird, der dann wiederum als Druckimpuls auf die im Reservoir 39 befindliche Flüssigkeit gelangt.

Am entgegengesetzten Ende des Kolbens 32 ist eine Rückrutschsperre 35 vorgesehen, die bewirkt, daß der Kolben 32 nur in eine Richtung derart wandern kann, daß das Volumen des Flüssigkeitsreservoirs 39 verringert wird. Der Hammer 33 ist aus einem magnetisierbaren Material, während alle anderen Bauteile dieser Anordnung unmagnetisierbar sind.

Rings um den Zylinder 31 ist eine Spule 36 angeordnet, deren Wicklungsenden mit dem Ausgang eines Leistungsverstärkers 60 in Verbindung stehen. Wenn der Verstärker 60 entsprechend angesteuert wird, fließt eine Strom durch die Spule 36 und beschleunigt dadurch den Hammer 33 in Richtung auf den Kolben 32, auf den er auftrifft. Bei einer entsprechenden impulsartigen Ansteuerung baut sich das Magnetfeld bereits wieder ab, wenn der Hammer 33 kurz vor dem Auftreffen ist.

Die Ansteuerung des Leistungsverstärkers 60 erfolgt mittels einer Steuerschaltung 50, die eingangsseitig einen Steuergenerator 51 aufweist, der im wesentlichen rechteckförmige Impulse erzeugt, deren Dauer über ein erstes Einstellorgan 52 und deren Wiederholfrequenz durch ein zweites Einstellorgan 53 eingestellt werden. Mit 54 ist ein Startschalter bezeichnet, der die vom Generator 51 erzeugten (hinsichtlich Dauer und Frequenz definierten) Impulse einem Zähler 55 zuführt, dessen Inhalt einem Komparator 56 über entsprechende Verbindungsleitungen mitgeteilt wird. Der Komparator 56 vergleicht den Inhalt des Zählers 55 mit einem voreingestellten Wert aus einem Vorwähler 57. Sobald der Wert des Zählers 55 und der Wert des Vorwählers 57 übereinstimmen, wird das Ausgangssignal aus dem Generator, das bis zum Gleichstand der beiden Zahlen durch ein NAND-Gatter 58 auf einen Eingang eines Verstärkers 59 gelangte, gesperrt. Dies bedeutet, daß nach Drücken der Taste 54 (ein dem Fachmann geläufiges Zurücksetzen des Zählers 55 erfolgt gleichzeitig) eine durch den Vorwähler 57 vorbestimmte Anzahl von Impulsen zum Eingang des Verstärkers 59 und mit einer, durch eine Amplitudeneinstellung 61 vorgewählte Amplitude auf den Eingang des Leistungsverstärkers 60 gelangt, der wiederum einen dem Eingangssignal entsprechenden Strom durch die Spule 36 fließen läßt. Man kann also über eine Steuerung der elektrischen Größen bzw. eine Steuerung der den Kolben 32 beschleunigenden Parameter sowie die Anzahl der Einzelstöße, welche der Kolben 32 erfährt, festlegen, welche Flüssigkeitsmenge (in Einzel-Quanten) wie tief injiziert wird.

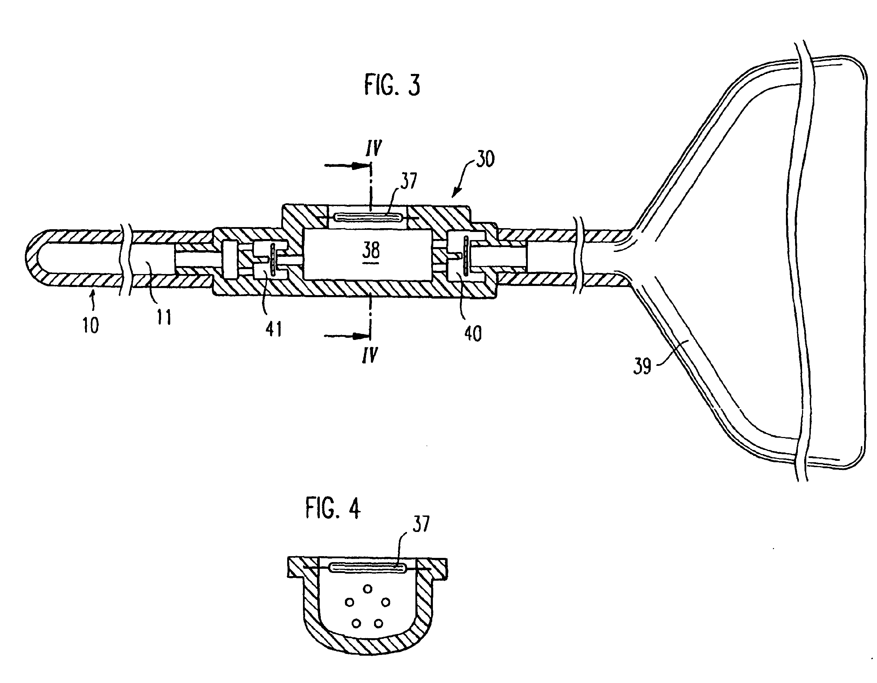

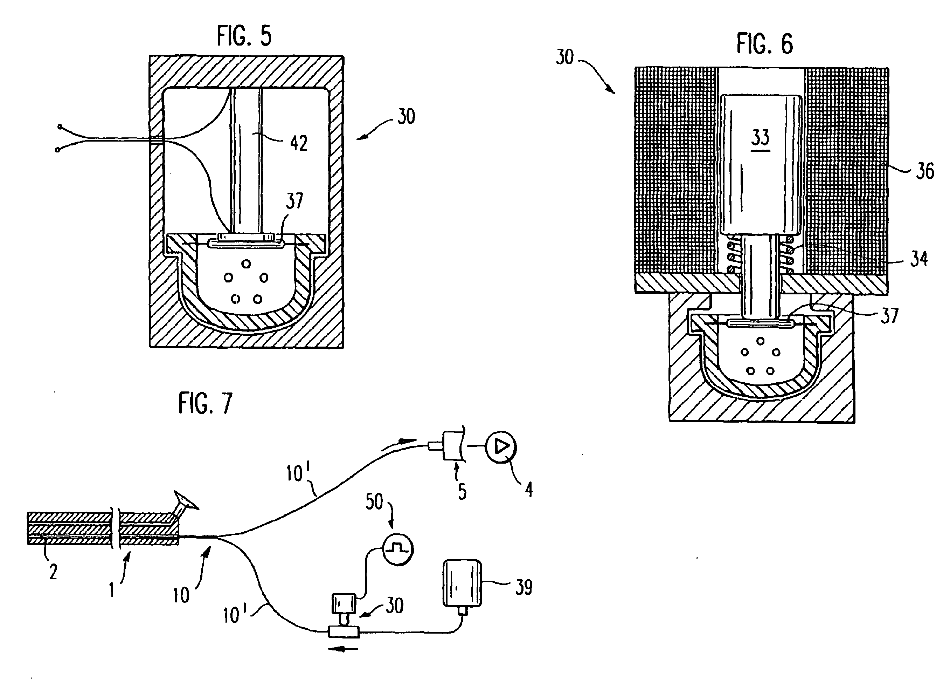

Bei der in Fig. 3 gezeigten Variante ist kein Kolben, sondern eine Membran 37 in der Pumpeinrichtung 30 vorgesehen. Diese Membran schließt einen Pumpraum 38 ab, der über ein erstes Rückschlagventil 40 mit einem Flüssigkeitsreservoir 39 und über ein zweites Rückschlagventil 41 mit dem Lumen 11 eines Schlauches 10 in Verbindung steht. Die Rückschlagventile 40 und 41 sind hierbei derart ausgestaltet, daß Flüssigkeit vom Flüssigkeitsreservoir 39 zum Lumen 11 des Schlauches 10 aber nicht umgekehrt strömen kann. Wie in den Fig. 3 und 4 angedeutet, bildet die Pumpeinrichtung 30 zusammen mit dem Flüssigkeitsreservoir 39 und dem Schlauch 10 einen insgesamt handhabbaren Körper, eben ein Einweg-System. Um nun die erwähnten Druckstöße abgeben zu können, muß die Membran 37 bewegt werden. Hierfür kann beispielsweise ein Piezoantrieb 42 gemäß Fig. 5 Verwendung finden, wobei aufgrund der sehr hohen Grenzfrequenz eines solchen Systems kein "Schlag" im eigentlichen Sinn erfolgen muß, da sich der Piezokristall bei einer entsprechend steilen Impulsanstiegsflanke der ihm zugeführten elektrischen Leistung sehr schnell ausdehnt und über die Membran 37 der Flüssigkeit im Pumpraum 38 einen entsprechenden Druckimpuls mitteilen kann. Ein entsprechend ausgebildetes elektrodynamisches System wäre hierzu ebenfalls in der Lage.

Bei dem in Fig. 6 gezeigten Antrieb handelt es sich wieder um ein elektromagnetisches Antriebssystem ähnlich dem nach Fig. 2, bei welchem ein Hammer 33 entgegen der Kraft einer Feder 34 durch die elektromagnetische Kraft einer Spule 36 beschleunigt wird und auf die Membran 37 auftrifft, so daß ein Druckimpuls in die Flüssigkeit geleitet wird, die sich im Pumpraum 38 unter der Membran 37 befindet. Die Ansteuerung der in den Fig. 5 und 6 gezeigten Ausführungsformen der Erfindung kann durch eine Steuerschaltung 50 erfolgen, wie sie anhand der Fig. 2 beschrieben wurde.

Das Gesamtsystem sieht in einer bevorzugten Ausführungsform der Erfindung nun so aus, wie in Fig. 7 gezeigt. Mit der Bezugsziffer 1 ist ein Endoskop angedeutet, durch dessen Arbeitskanal 2 der Schlauch 10 geschoben ist. Der Schlauch 10 ist in diesem Fall zweilumig und teilt sich in zwei Einzelabschnitte 10' und 10" (mit jeweils einem Lumen), wobei der eine Abschnitt 10' in eine Pumpeinrichtung 30 führt, welche Flüssigkeit aus einem Reservoir 39 ansaugt und - gesteuert durch eine Steuereinrichtung 50 - eine entsprechende Anzahl von Flüssigkeits-Quanten in ein Gewebe injizieren kann. Der andere Schlauchabschnitt 10" steht über eine Steckverbindung 5 mit einer Saugquelle 4 in Verbindung. Dadurch wird im Endabschnitt des Schlauches 10 befindliche Flüssigkeit, die gegebenenfalls über das Ende des Schlauches 10 austreten könnte, wenn das Endoskop 1 beim Hantieren verbogen wird und sich so das Volumen des Schlauches 10 ändert, abgesaugt.

### Bezugszeichenliste

- 1: Endoskop
- 2: Arbeitskanal
- 4: Saugquelle
- 5: Steckverbindung
- 10: Schlauch
- 11: Lumen
- 12: Lumen
- 13: Düse
- 14: Endabschnitt
- 20: Zieleinrichtung
- 21: Abstandsfinger
- 22: Lichtleiter
- 23: Markierung
- 30: Pumpeinrichtung
- 31: Zylinder
- 32: Kolben
- 33: Hammer
- 34: Feder
- 35: Rück-Sperre
- 36: Spule
- 37: Membran
- 38: Pumpraum
- 39: Flüssigkeitsreservoir
- 40: erstes Rückschlagventil
- 41: zweites Rückschlagventil
- 42: Piezoantrieb
- 50: Steuerschaltung
- 51: Steuergenerator
- 52: Dauer-Einstellung
- 53: Wiederholfrequenz-Einstellung
- 54: Startschalter
- 55: Zähler
- 56: Komparator
- 57: Vorwähler
- 58: NAND-Gatter
- 59: Einstell-Verstärker
- 60: Leistungs-Verstärker
- 61: Amplitudeneinstellung

## Patentansprüche

1. Endoskopische Injektionsvorrichtung zum Injizieren mindestens einer Lösung, Suspension, Emulsion oder dergleichen Flüssigkeit in ein Gewebe in einem Hohlorgan, einer Körperhöhle oder einer künstlichen Höhle in einem menschlichen oder tierischem Körper, umfassend
einen Schlauch (10) mit einem distalen sowie einem proximalen Ende;
eine Pumpeinrichtung (30), die mit dem proximalen Ende des Schlauches (10) in dichter Verbindung steht und derart ausgebildet ist, dass auf ein Steuersignal einer Steuerschaltung (5) hin eine definierte Flüssigkeitsmenge mit definiert einstellbaren hydraulischen Parametern, wie Druck, Druckanstiegsgeschwindigkeit, Dauer oder Geschwindigkeit aus dem distalen Schlauchende mit einer solchen Energie ausgestoßen wird, dass die Flüssigkeit in das Gewebe eindringt, **dadurch gekennzeichnet, daß** der Schlauch durch einen Arbeitskanal (2) eines Endoskops (1) derart hindurchführbar oder in ein Endoskop (1) derart eingebaut ist, dass das distale Ende des Schlauches (10) aus einem distalen Ende des Endoskops (1) hervortritt.

2. Vorrichtung nach Anspruch 1,
**dadurch gekennzeichnet, dass** der Durchmesser des distalen Endes des Schlauches (10) derart geformt oder mit einer Düse (13) eines derartigen Durchmessers versehen ist, dass sich das im Gewebe beim Injizieren entstehende Loch schließt, ohne im wesentlichen die Injizierte Flüssigkeit zuvor zu entlassen.

3. Vorrichtung nach einem der Ansprüche 1 oder 2,
**dadurch gekennzeichnet, dass** die Pumpeinrichtung (30) zur Injektion einer gewünschten Flüssigkeitsmenge in einer Vielzahl von nacheinander injizierten kleinen Einzelvolumina ausgebildet ist.

4. Vorrichtung nach einem der Ansprüche 1 bis 3,
**dadurch gekennzeichnet, daß**
am distalen Ende des Schlauches (10) Ziel- und/oder Positioniereinrichtungen (20) vorgesehen sind, um das distale Ende in einem vorbestimmten Abstand vom Gewebe und/oder einer vorbestimmten Richtung zum Gewebe zu halten.

5. Vorrichtung nach Anspruch 4,
**dadurch gekennzeichnet, dass** die Ziel- und/oder Positioniereinrichtungen (20) zum Injizieren der Flüssigkeit unter einem Winkel von etwa 30° bis 60° in das Gewebe ausgebildet ist.

6. Vorrichtung nach einem der Ansprüche 1 bis 5,
**dadurch gekennzeichnet, daß**
der Schlauch (10) mindestens in seinem distalen Abschnitt mehrere, insbesondere zwei Lumina (11, 12) aufweist, und
daß die Pumpeinrichtung (30) mehrere Einzel-Pumpen umfaßt, um mehrere, insbesondere zwei Flüssigkeiten zu deren Vermischung zu injizieren.

7. Vorrichtung nach einem der Ansprüche 1 bis 6,
**dadurch gekennzeichnet, daß**
der Schlauch (10) ein Gas- und/oder Flüssigkeitsrückführungslumen (12) umfaßt, das derart beim distalen Ende des Schlauches (10) in den Schlauch (10) mündet, daß im Schlauch (10) enthaltene Flüssigkeit am Austreten aus dem distalen Ende gehindert wird.

8. Vorrichtung nach Anspruch 7,
**dadurch gekennzeichnet, daß**
eine Saugeinrichtung (4) zum Absaugen von Flüssigkeit aus dem Flüssigkeitsrückführungslumen (12) vorgesehen ist.

9. Vorrichung nach einem der Ansprüche 1 bis 8,
**dadurch gekennzeichnet, daß**
der Schlauch (10) bei seinem distalen Ende einen einlumigen Abschnitt (14) oder eine Düse (13) aufweist.

10. Vorrichtung nach einem der Ansprüche 1 bis 9,
**dadurch gekennzeichnet, daß**
der Schlauch (10) aus einem derartigen Material und derart drucksteif ausgebildet und/oder mit Versteifungseinrichtungen derart versehen ist, daß ein in sein proximales Ende eingeführter Druckpuls im wesentlichen ungedämpft oder unverändert aus dem distalen Ende austritt.

11. Vorrichtung nach einem der Ansprüche 1 bis 10,
**dadurch gekennzeichnet, dass** der Schlauch (10) und ein Flüssigkeitsreservoir (39) zum Speichern großer, während der Dauer einer längern Anwendung oder Operation zu injizierender Flüssigkeitsmengen als einheitlich handhabbare sterile Injektionseinheit ausgebildet ist.

12. Vorrichtung nach Anspruch 11,
**dadurch gekennzeichnet, daß**
die Injektionseinheit als nur einmal zu verwendendes Teil ausgebildet ist.

13. Vorrichtung nach einem der Ansprüche 11 oder 12,
**dadurch gekennzeichnet, daß**
die Injektionseinheit die Pumpeinrichtung (30) mindestens teilweise mitumfaßt.

14. Vorrichtung nach Anspruch 13,
**dadurch gekennzeichnet, daß**
die Pumpeinrichtung (30) ein Kolben (32), eine Membran (37) oder dergleichen bewegbares Element als Druckerzeugungsteil umfaßt und durch eine gesonderte, vorzugsweise wiederverwendbare Antriebseinrichtung (36, 42) mit einer einstellbaren Pumpkraft beaufschlagbar ist.

## Claims

1. Endoscopic injection device for injecting at least one solution, suspension, emulsion or similar fluid into a tissue in a hollow organ, a body cavity or an artificial cavity in a human or animal body, comprising
a tube (10) with a distal and a proximal end;
a pump mechanism (30), which is impermeably connected to the proximal end of the tube (10) and is configured in such a way that, in response to a control signal from control circuit (5), a defined quantity of fluid is discharged from the distal end of the tube with hydraulic parameters such as pressure, rate of pressure rise, duration or velocity adjustment to specified values to provide sufficient energy for the fluid to penetrate the tissue,
**characterised in that**
the tube can be guided through a working channel (2) of an endoscope (1) or is integrated into an endoscope (1) in such a way that the distal end of the tube (10) emerges from a distal end of the endoscope (1).

2. Device according to Claim 1,
**characterised in that** the diameter of the distal end of the tube (10) is shaped in such a way or is provided with a nozzle (13) of such diameter that the hole created in the tissue upon injection closes up without substantially allowing the injected fluid to escape prior to this.

3. Device according to one of Claims 1 or 2,
**characterised in that** the pump mechanism (30) is configured to inject a desired quantity of fluid in a plurality of small individual volumes injected in succession.

4. Device according to one of Claims 1 to 3,
**characterised in that** aiming and/or positioning elements (20) are provided at the distal end of the tube (10) to keep the distal end at a predetermined distance from the tissue and/or in a predetermined direction relative to the tissue.

5. Device according to Claim 4,
**characterised in that** the aiming and/or positioning elements (20) are configured to inject the fluid into the tissue at an angle of around 30° to 60°.

6. Device according to one of Claims 1 to 5,
**characterised in that** the tube (10) has several, in particular two, lumina (11, 12) at least in its distal section and that the pump mechanism (30) comprises several individual pumps in order to inject several, in particular two, fluids to mix them.

7. Device according to one of Claims 1 to 6,
**characterised in that** the tube (10) comprises a gas and/or fluid return lumen (12), which joins the tube (10) at the distal end of the tube (10) in such a way that fluid contained in the tube (10) is prevented from emerging from the distal end.

8. Device according to Claim 7,
**characterised in that** a suction element (4) is provided for drawing off fluid from the fluid return lumen (12).

9. Device according to one of Claims 1 to 8,
**characterised in that** the tube (10) has a single-lumen section (14) or a nozzle (13) at its distal end.

10. Device according to one of Claims 1 to 9,
**characterised in that** the tube (10) is configured of such material and is so rigid in compression and/or provided with reinforcing elements in such a way that a pressure pulse introduced into its proximal end emerges substantially unattenuated or unchanged from the distal end.

11. Device according to one of Claims 1 to 10,
**characterised in that** the tube (10) and a fluid reservoir (39) for storing large quantities of fluid to be injected throughout the duration of a longer application or operation are configured as a uniformly manageable sterile injection unit.

12. Device according to Claim 11,
**characterised in that** the injection unit is configured as a part that is to be used only once.

13. Device according to one of Claims 11 or 12,
**characterised in that** the injection unit also comprises at least part of the pump mechanism (30).

14. Device according to Claim 13,
**characterised in that** the pump mechanism (30) comprises a piston (32), a membrane (37) or similar moveable element as a pressure generating part and can be subjected to an adjustable pumping force by means of a separate, preferably reusable, drive mechanism (36, 42).

## Revendications

1. Dispositif d'injection endoscopique pour injecter au moins une solution, une suspension, une émulsion ou un liquide similaire dans un tissu d'un organe creux, dans une cavité corporelle ou dans une cavité artificielle d'un organisme humain ou animal, comprenant :
une tubulure (10) avec une extrémité distale et une extrémité proximale ;
un système de pompage (30) qui est en liaison étanche avec l'extrémité proximale de la tubulure (10) et qui est configuré de telle façon que, en réponse à un signal de commande d'un circuit de commande (5), une quantité de liquide définie dont les paramètres hydrauliques tels que la pression, la vitesse d'élévation de la pression, la durée ou la vitesse sont ajustables de manière définie est expulsée par l'extrémité distale de la tubulure avec une énergie telle que le liquide pénètre dans le tissu, **caractérisé en ce que** la tubulure peut être introduite dans un conduit de travail (2) d'un endoscope (1) ou bien incorporée dans un endoscope (1) de telle façon que l'extrémité distale de la tubulure (10) sort par une extrémité distale de l'endoscope (1).

2. Dispositif selon la revendication 1, **caractérisé en ce que** le diamètre de l'extrémité distale de la tubulure (10) est formé de telle façon ou équipé d'une buse (13) ayant un diamètre tel que le trou formé dans le tissu lors de l'injection se referme pratiquement sans laisser ressortir auparavant le liquide injecté.

3. Dispositif selon l'une des revendications 1 ou 2, **caractérisé en ce que** le système de pompage (30) est conçu pour injecter une quantité de liquide voulue en plusieurs petits volumes individuels injectés à la suite les uns des autres.

4. Dispositif selon l'une des revendications 1 à 3, **caractérisé en ce qu'**il est prévu à l'extrémité distale de la tubulure (10) des systèmes d'orientation et/ou de positionnement (20) qui servent à maintenir l'extrémité distale à une distance prédéterminée du tissu et/ou dans une direction prédéterminée par rapport au tissu.

5. Dispositif selon la revendication 4, **caractérisé en ce que** les systèmes d'orientation et/ou de positionnement (20) sont configurés pour injecter le liquide suivant un angle d'environ 30° à 60° dans le tissu.

6. Dispositif selon l'une des revendications 1 à 5, **caractérisé en ce que** la tubulure (10) présente au moins dans sa partie distale plusieurs, en particulier deux lumières (11, 12) et **en ce que** le système de pompage (30) comprend plusieurs pompes individuelles afin d'injecter plusieurs, en particulier deux liquides dans le but de les mélanger.

7. Dispositif selon l'une des revendications 1 à 6, **caractérisé en ce que** la tubulure (10) comprend une lumière de retour de gaz et/ou de liquide (12) qui débouche dans la tubulure (10) au niveau de l'extrémité distale de celle-ci afin d'empêcher le liquide contenu dans la tubulure (10) de ressortir par l'extrémité distale.

8. Dispositif selon la revendication 7, **caractérisé en ce qu'**il est prévu un système d'aspiration (4) pour aspirer du liquide dans la lumière de retour de liquide (12).

9. Dispositif selon l'une des revendications 1 à 8, **caractérisé en ce que** la tubulure (10) présente à son extrémité distale un segment à une seule lumière (14) ou une buse (13).

10. Dispositif selon l'une des revendications 1 à 9, **caractérisé en ce que** la tubulure (10) est faite d'un matériau tel et présente une rigidité à la pression telle et/ou est munie de dispositifs raidisseurs tels qu'une impulsion de pression introduite par son extrémité proximale ressort par l'extrémité distale pratiquement sans être amortie ou modifiée.

11. Dispositif selon l'une des revendications 1 à 10, **caractérisé en ce que** la tubulure (10) et un réservoir de liquide (39) permettant de stocker de grandes quantités de liquide à injecter au cours d'une utilisation ou opération de longue durée sont réalisés sous la forme d'un ensemble d'injection stérile qui peut être manipulé d'un seul tenant.

12. Dispositif selon la revendication 11, **caractérisé en ce que** l'ensemble d'injection est réalisé sous la forme d'un élément à usage unique.

13. Dispositif selon l'une des revendications 11 ou 12, **caractérisé en ce que** l'ensemble d'injection intègre au moins en partie le système de pompage (30).

14. Dispositif selon la revendication 13, **caractérisé en ce que** le système de pompage (30) comprend un piston (32), une membrane (37) ou un élément mobile similaire en tant qu'élément générateur de pression et **en ce qu'**une force de pompage réglable peut être appliquée par le biais d'un système d'entraînement (36, 42) séparé, de préférence réutilisable.
